Europäisches Patentamt

(19) European Patent Office

Office européen des brevets

(11) Numéro de publication: **0 258 073**
A1

# (12) DEMANDE DE BREVET EUROPEEN

(21) Numéro de dépôt: 87401335.2

(22) Date de dépôt: 15.06.87

(51) Int. Cl.⁴: **A 61 M 5/20**
A 61 M 5/14

(30) Priorité: 16.06.86 FR 8608668
09.01.87 FR 8700176

(43) Date de publication de la demande:
02.03.88 Bulletin 88/09

(84) Etats contractants désignés:
BE CH DE ES FR GB IT LI LU NL

(71) Demandeur: **Pistor, Michel**
**87, boulevard Suchet**
**F-75016 Paris (FR)**

(72) Inventeur: **Pistor, Michel**
**87, boulevard Suchet**
**F-75016 Paris (FR)**

**Muis, Pascal**
**18, rue Bourdelle**
**F-95000 Pontoise (FR)**

(74) Mandataire: **Portal, Gérard et al**
**Cabinet Beau de Loménie 55, rue d'Amsterdam**
**F-75008 Paris (FR)**

Le titre de l'invention a été modifié (Directives relatives à l'examen pratiqué à l'OEB, A-III, 7.3)

(54) **Dispositif d'injection transcutanée portatif.**

(57) Dispositif thérapeutique comprenant un élément support (8) d'une membrane déformable (6) qui, à l'état déformé, emmagasine une énergie suffisante pour réaliser l'injection d'une solution liquide d'au moins un agent thérapeutique introduit par injection forcée par un organe de remplissage formé par exemple par un élément d'obturation perforable (18) introduit dans un orifice (16) de l'élément support (8) et qui provoque une déformation par gonflement ou renflement de la membrane (6).

On obtient ainsi l'injection transcutanée progressive sous la pression de l'injection provoquée par la membrane (6) qui reprend progressivement sa forme initiale, par l'organe d'injection (20), d'une manière tout à fait simple.

Fig-1

## Description

Procédé de fabrication d'un dispositif thérapeutique d'injection transcutanée, portable, totalement autonome et dispositif thérapeutique d'iniection transcutanée, portable, totalement autonome

L'invention concerne essentiellement un procédé de fabrication d'un dispositif thérapeutique d'injection transcutanée, portable, totalement autonome, ainsi qu'un dispositif thérapeutique d'injection transcutanée, portable, totalêment autonome.

On connaît de nombreux dispositifs thérapeutiques transdermiques pour l'apport pendant une période de temps prolongée d'au moins un agent à activité thérapeutique, se présentant en général sous forme d'une solution liquide.

Les dispositifs thérapeutiques transdermiques connus comprennent un élément formant réservoir pourvu de moyens permettant l'apport de l'agent à activité thérapeutique lors de l'application du dispositif sur le derme intact d'un être viv-nt. On peut citer les documents ALZA FR-2 143 564 ; FR-A-2 508 318 ; FR-A-2 158 201 ; FR-A-2 121 831 ; FR-A-2 299 853 ; FR-A-2 527 450 ; FR-A-2 545 357 ; EP-A1-0 010 876 ; EP-A3-0 033 615 ; EP-A2-0 052 916.

Tous les dispositifs connus sont appliqués sur la peau intacte et ne permettent un traitement thérapeutique transcutané efficace que pour des agents thérapeutiques en nombre limité qui sont capables de traverser la barrière cutanée.

D'autre part, on connaît par des brevets antérieurs du demandeur FR-A-2 232 331 ; FR-A-2 309 242 ; FR-A-2 348 709 et FR-A-2 390 175 divers dispositifs adaptés pour des traitements loco-régionaux (T.L. R.). Ces derniers dispositifs permettent de franchir la barrière cutanée par l'utilisation d'aiguilles ou pointes intradermiques ou même sous-cutanées. Ces dispositifs réalisent une injection des substances de traitement de manière pratiquement instantanée ou en quelques secondes, cette injection étant effectuée par le praticien.

Les présents inventeurs ont maintenant pu constater que l'action de ces substances pouvait être améliorée de manière inattendue par l'injection de ces substances pendant une période de temps prolongée de quelques minutes, si possible de quelques heures, et encore mieux pendant un ou plusieurs jours.

Par ailleurs, les présents inventeurs ont pu constater que l'action des dispositifs tels que décrits dans les brevets ALZA précités 2 143 564 ; 2 508 318 ou 2 158 201, ou encore 2 299 853 ou encore 2 527 450 pouvait être grandement améliorée de manière inattendue en réalisant à l'aide d'une aiguille ou pointe stérile un piquetage de la peau préalablement à l'application dudit dispositif précité.

De ce fait, la présente invention a donc pour but de résoudre un nouveau problème technique consistant en la fourniture d'un dispositif thérapeutique d'injection transcutanée utilisable pour n'importe quel type d'agent à activité thérapeutique pendant une période de temps prolongée, ce dispositif étant portable par le patient d'une manière totalement autonome, avec de préférence possibilité de modification à tout moment de la composition de l'agent thérapeutique, afin d'adapter la composition d'agent(s) thérapeutique(s) au traitement nécessité par l'état du patient, en obtenant ainsi un traitement personnalisé.

La présente invention a encore pour but de résoudre un autre problème technique consistant en la fourniture d'une solution de conception particulièrement simple ne mettant en oeuvre qu'un minimum de pièces pour constituer le dispositif thérapeutique d'injection transcutanée, permettant avantageusement de préserver le caractère stérile nécessaire de tous dispositifs thérapeutiques au cours du temps, ou permettant une stérilisation de celui-ci préalablemênt à l'usage.

La présente invention a encore pour but de résoudre un nouveau problème technique consistant en la fourniture d'un dispositif thérapeutique d'injection transcutanée permettant l'injection, d'une manière extrêmement simple, d'une manière automatique et avec le minimum d'interventions de la part du praticien.

La présente invention a encore pour but de résoudre un nouveau problème technique consistant en la fourniture d'un dispositif thérapeutique d'injection transcutanée dont la conception est tellement simple qu'il peut être fabriqué à un coût si faible qu'il peut être jetable.

Tous ces problèmes techniques sont résolus pour la première fois par la présente invention, d'une manière particulièrement simple et économique.

Ainsi, selon un premier aspect, la présente invention fournit un procédé de fabrication d'un dispositif thérapeutique d'injection transcutanée, portable, totalement autonome, comprenant un élément formant réservoir pour la réception d'une solution liquide d'au moins un agent à activité thérapeutique, caractérisé en ce qu'on prévoit au moins un organe d'injection transcutanée, par exemple une aiguille ou une pointe, et des moyens pour provoquer l'injection transcutanée de ladite solution depuis ledit réservoir, de préférence on prévoit une membrane déformable comme moyen provoquant l'injection de la solution depuis ledit réservoir, et on monte ladite membrane sur ou dans ledit dispositif thérapeutique de manière à permettre un mouvement de la membrane réduisant le volume du réservoir d'agent thérapeutique, notamment lorsqu'on exerce sur la membrane, en direction de l'organe d'injection, une pression supérieure à la pression régnant dans le réservoir, pour provoquer alors ladite injection transcutanée.

Selon une caractéristique particulière du procédé de fabrication selon l'invention, on monte la membrane déformable à l'état non déformé, en l'absence d'agent thérapeutique, de sorte que ledit réservoir est d'un volume réduit, voire sensiblement nul, cette membrane étant montée de manière à pouvoir être déformée à un moment quelconque avant l'injection transcutanée, avantageusement par l'introduction forcée de la solution liquide d'agent thérapeutique dans le réservoir, cette déformation étant prévue pour permettre d'emmagasiner une quantité d'énergie dans la membrane suffisante pour permettre l'injection progressive transcutanée de ladite solution d'agent à activité thérapeutique, lorsque la membrane reprend progressivement sa forme initiale non déformée.

Selon une autre réalisation particulière du procédé de fabrication selon l'invention, on prévoit un dispositif thérapeutique comprenant une cavité dans laquelle on dispose la membrane déformable précitée de manière à subdiviser ladite cavité en deux compartiments distincts, non communicants, un compartiment constituant le réservoir de solution liquide d'agent thérapeutique précitée et l'autre compartiment étant prévu pour pouvoir exercer contre la membrane, de préférence à l'aide d'un gaz introduit sous pression, une pression supérieure à la pression régnant dans le compartiment contenant la solution liquide d'agent thérapeutique.

Selon un deuxième aspect, la présente invention fournit également un dispositif thérapeutique d'injection transcutanée, portable, totalement autonome, comprenant un élément formant réservoir pour la réception d'une solution liquide d'au moins un agent à activité thérapeutique, caractérisé en ce qu'il est prévu pour recevoir au moins un organe d'injection transcutanée, par exemple une aiguille ou une pointe, et des moyens pour provoquer l'injection de ladite solution liquide depuis ledit réservoir, de préférence il comprend une membrane déformable comme moyen provoquant l'injection de ladite solution ; et des moyens permettant un déplacement de la membrane réduisant le volume dudit réservoir en provoquant ainsi une injection transcutanée par ledit organe d'injection.

Selon une caractéristique particulière du dispositif thérapeutique selon l'invention, celui-ci est caractérisé en ce que la membrane déformable est capable d'être déformée suffisamment pour emmagasiner une énergie suffisante pour injecter ladite solution liquide d'agent thérapeutique depuis ledit réservoir d'une manière progressive, lorsque la membrane reprend progressivement sa forme initiale non déformée.

Selon une autre caractéristique particulière de l'invention, la membrane déformable est déformée à un moment quelconque avant l'injection, de préférence juste avant l'injection, encore de préférence cette déformation de la membrane est obtenue par l'introduction forcée de la solution liquide d'agent à activité thérapeutique dans ledit réservoir.

Selon encore une autre caractéristique particulière de l'invention, la membrane précitée constitue au moins une partie du réservoir.

Selon une autre caractéristique de l'invention, le dispositif est caractérisé en ce qu'il est pourvu d'un organe de remplissage du réservoir qui peut, selon une première variante, être formé par une matière perforable ou, selon une seconde variante, comprendre une valve, avantageusement un clapet antiretour.

Selon un mode de réalisation particulièrement avantageux, la membrane déformable précitée est montée sur un élément support de membrane qui comporte une partie de paroi définissant, avec la membrane, le réservoir précité.

Selon un mode de réalisation particulièrement avantageux, l'élément support a en coupe au moins partiellement une forme sensiblement cylindrique creuse.

Avantageusement, cet élément support peut comporter à une extrémité un épaulement annulaire radialement saillant vers l'extérieur contre lequel est maintenu solidement le bord de la membrane par un moyen de maintien approprié tel qu'une bague de serrage.

Selon un autre mode de réalisation de l'invention, le dispositif thérapeutique précité comprend une cavité dans laquelle est montée la membrane précitée de manière à subdiviser cette cavité en deux compartiments distincts non communicants dont l'un constitue le réservoir de solution liquide d'agent thérapeutique précitée et l'autre compartiment comprend des moyens prévus pour pouvoir exercer contre la membrane, de préférence à l'aide d'un gaz introduit sous pression, une pression supérieure à la pression régnant dans le réservoir rempli de ladite solution liquide d'agent thérapeutique.

Selon un mode de réalisation également avantageux, on peut prévoir que l'organe d'injection transcutanée n'est pas monté sur dispositif thérapeutique lors de sa fabrication mais seulement par le praticien au moment de l'emploi de celui-ci. Pour ce faire, on peut prévoir que le dispositif thérapeutique comprend un moyen de fixation de l'organe d'injection transcutanée qui, selon une variante de réalisation, peut être formé par un orifice communiquant avec le réservoir de solution liquide, normalement obturé par des moyens d'obturation comprenant de préférence un élément d'obturation perforable pouvant être constitué par un simple bouchon, par exemple en caoutchouc, qui peut être disposé de manière à être séparable à volonté du dispositif ou être incorporé à demeure dans le dispositif, comme cela peut être aussi le cas pour l'organe de remplissage constituant des moyens d'introduction de la solution liquide d'agent thérapeutique précitée.

Selon un autre mode de réalisation particulier, l'organe de remplissage est avantageusement disposé sur la face du dispositif comportant l'organe d'injection transcutanée.

On comprend ainsi qu'il est possible de remplir au préalable le réservoir avec une solution liquide d'au moins un agent thérapeutique et qu'ensuite le praticien peut modifier à volonté la composition de l'agent thérapeutique grâce à l'introduction, par l'organe de remplissage précité, d'au moins un autre agent thérapeutique en adaptant ainsi la composition au traitement particulier nécessité par l'état du patient.

En outre, il est également possible de laisser le réservoir vide de sorte que le praticien effectue lui-même le remplissage avec le mélange de son choix et de sa composition au dernier moment, par l'organe de remplissage selon la présente invention.

Par ailleurs, grâce à la conception particulièrement simple du dispositif thérapeutique selon l'invention, l'injection de la solution liquide d'agent thérapeutique peut être réalisée progressivement dans le temps d'une manière extrêmement simple, avec un minimum d'interventions de la part du praticien, en toute sécurité, en conservant le caractère stérile du dispositif.

On comprend ainsi que l'on obtient bien tous les avantages techniques précédemment énoncés, d'une manière totalement inattendue, d'une façon très simple et très aisée à mettre en oeuvre.

L'utilisation préférée du dispositif selon l'invention concerne les traitements loco-régionaux (T.L.R.).

Par ailleurs, par l'expression "solution liquide", on entend incorporation de l'agent thérapeutique dans une composition en solution suffisamment fluide pour permettre l'écoulement ou l'injection.

D'autres buts, caractéristiques et avantages de l'invention apparaîtront clairement à la lumière de la description explicative qui va suivre faite en référence aux dessins annexés représentant dix modes de réalisation de l'invention donnés simplement à titre d'illustration et qui ne sauraient donc en aucune façon limiter la portée de l'invention. Dans les dessins :

- la figure 1 représente un premier mode de réalisation d'un dispositif thérapeutique d'injection transcutanée selon l'invention, comprenant une membrane déformable, représentée à l'état gonflé par des traits interrompus ;

- la figure 2 représente un deuxième mode de réalisation d'un dispositif thérapeutique selon l'invention permettant d'utiliser des aiguilles d'injection transcutanée standards du commerce, constituant une variante du dispositif thérapeutique de la figure 1 ;

- la figure 3 représente un troisième mode de réalisation d'un dispositif thérapeutique selon l'invention, également en coupe transversale très agrandie ;

- la figure 4 représente une coupe partielle du dispositif de la figure 3 mais selon une variante de réalisation de celui-ci selon laquelle l'organe de remplissage est monté à demeure dans le dispositif ;

- la figure 5 représente une variante de réalisation du dispositif thérapeutique de la figure 3 selon laquelle l'organe d'injection transcutanée est monté sur le dispositif par le praticien au moment de l'emploi ;

- la figure 6 représente un sixième mode de réalisation du dispositif thérapeutique selon l'invention, selon une variante de réalisation du dispositif de la figure 5 ;

- la figure 7 représente un septième mode de réalisation du dispositif thérapeutique selon l'invention constituant une variante du mode de réalisation de la figure 5 ;

- la figure 7a représente une vue de détail agrandie de l'organe de remplissage du mode de réalisation de la figure 7, comportant une valve formant clapet antiretour ;

- la figure 8 représente un huitième mode de réalisation d'un dispositif thérapeutique selon l'invention, constituant une variante de réalisation de celui de la figure 3 ;

- la figure 9 représente un neuvième mode de réalisation d'un dispositif thérapeutique selon l'invention constituant une variante de réalisation de celui de la figure 8 ; et

- la figure 10 représente encore un dixième mode de réalisation d'un dispositif thérapeutique selon l'invention constituant une variante de réalisation de celui des figures 8 et 9.

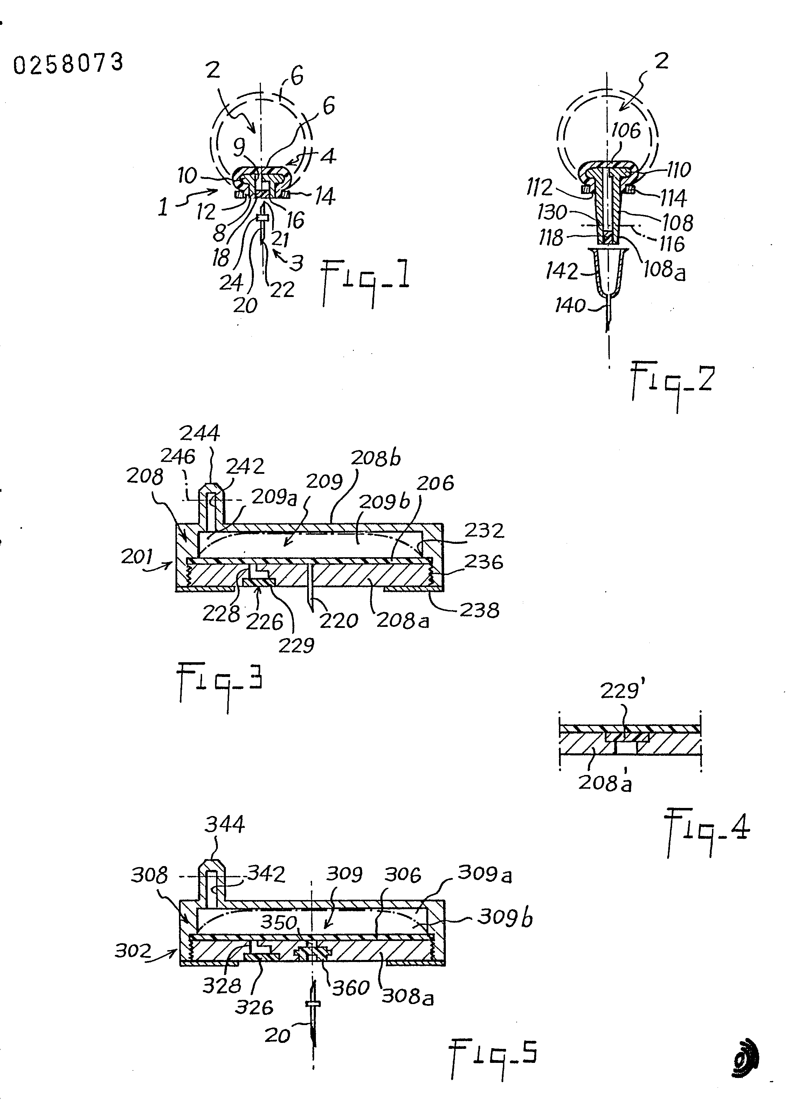

En référencê à la figure 1, on a représenté un premier mode de réalisation d'un dispositif thérapeutique selon l'invention représenté par le numéro de référence général 1, qui permet une injection transcutanée, est portable et totalement autonome. Ce dispositif comprend un élément formant réservoir représenté par le numéro de référence générale 2, pour la réception d'une solution liquide d'au moins un agent à activité thérapeutique, qui est prévu pour recevoir au moins un organe 3 d'injection transcutanée, par exemple une aiguille ou une pointe.

Ce dispositif comprend également des moyens 4 pour provoquer l'injection de la solution liquide depuis ledit réservoir 2.

Selon la présente invention, ce dispositif thérapeutique 1 est caractérisé en ce qu'il comprend une membrane déformable 6 comme moyen 4 provoquant l'injection de la solution, ainsi que des moyens permettant un déplacement de la membrane réduisant le volume du réservoir 2 en provoquant ainsi l'injection transcutanée.

Ces moyens qui permettent de réduire le volume du réservoir sont formés par un montage de la membrane 6 à l'état non déformé, le réservoir 2 étant vide, de sorte que le réservoir 2 est d'un volume réduit, voire sensiblement nul, comme représenté à la figure 1.

Selon une variante de réalisation avantageuse, la membrane déformable 6 forme au moins une partie du réservoir 2 de solution liquide d'agent thérapeutique comme cela est clairement visible à la figure 1.

Cette membrane déformable 6 est avantageusement montée sur un élément 8 support de membrane qui comporte une partie de paroi 9 définissant avec la membrane 6 le réservoir 2 précité.

Cet élément support 8 a en coupe au moins partiellement une forme sensiblement cylindrique creuse.

Selon une autre caractéristique avantageuse, cet élément support 8 comprend une extrémité coopérant avec la membrane 6, un épaulement annulaire 10 radialement saillant vers l'extérieur contre lequel est maintenu solidement le bord 12 de la membrane 6 par un moyen de maintien approprié 14 tel qu'une bague de serrage.

Selon ce premier mode de réalisation représenté à la figure 1, l'ouverture 16 de l'élément support 8 est obturée par un moyen d'obturation 18 qui est ici avantageusement perforable en étant constitué par un simple bouchon, par exemple en caoutchouc, comme cela est classique en matière médicale.

Cet élément d'obturation 18 perforable peut ainsi être perforé juste avant l'emploi pour permettre l'introduction par le praticien d'une solution liquide d'au moins un agent thérapeutique, à l'aide d'une seringue classique, ce qui va provoquer une déformation et un renflement de la membrane 6 qui va prendre la position renflée sensiblement sphérique représentée en traits interrompus à la figure 1.

Ensuite, le praticien peut introduire un organe d'injection sous-cutanée 3, par exemple, constitué par une aiguille creuse 20 qui est biseautée aux deux extrémités 21, 22, l'extrémité 21 étant par exemple enfoncée au travers de l'élément d'obturation 18 de manière à le perforer tandis que l'extrémité 22 est enfoncée dans la peau du patient, pour réaliser une injection transcutanée. On prévoit sur cette aiguille 20 un anneau d'arrêt 24 servant à arrêter l'insertion de l'aiguille 20 au travers de l'élément d'obturation 18.

On comprend ainsi que l'on obtient un dispositif thérapeutique d'une dimension extrêmement réduite, d'une conception extrêmement simple comportant un minimum de pièces, qui est parfaitement portable, totalement autonome, et qui permet une injection transcutanée d'une solution liquide d'au moins un agent thérapeutique pouvant être introduit au moment de l'emploi selon une formulation adaptée à volonté par le praticien permettant de réaliser un traitement personnalisé de ce patient.

En outre, lors de l'introduction de la substance liquide dans le réservoir 2, la déformation de la membrane 6 qui sera provoquée par l'introduction forcée de la solution liquide contenant le ou les agents thérapeutiques va provoquer le gonflement ou renflement de la membrane déformable 6, comme indiqué en traits interrompus à la figure 1, ce qui va emmagasiner dans la membrane une quantité d'énergie suffisante pour permettre l'injection progressive transcutanée de ladite solution lorsque la membrane va reprendre progressivement sa forme initiale non déformée.

On comprend qu'en réglant le caractère déformable, c'est-à-dire en pratique l'élasticité de la membrane 6, on pourra régler le temps d'injection de la solution liquide, ce temps pouvant donc être réglé de quelques minutes à plusieurs heures, voire un ou plusieurs jours.

Naturellement, ce dispositif thérapeutique peut être stérilisé à l'origine sans aucun problème. Il se présente sous la forme d'une pastille d'une dimension très réduite, d'un coût extrêmement modeste grâce à sa simplicité de conception.

Ce dispositif peut être pourvu, sur la surface destinée à être appliquée sur la peau, d'un adhésif protégé provisoirement par une couche de protection détachable, comme cela est bien connu, en particulier par les documents ALZA précités, afin de permettre l'adhésion parfaite sur la peau ou le derme d'un être vivant. Cet adhésif ne doit pas être nécessairement perméable, contrairement aux dispositifs antérieurement connus qui n'utilisaient pas d'aiguille assurant le franchissement de la barrière cutanée.

En référence à la figure 2, on a représenté un deuxième mode de réalisation constituant une variante de réalisation de celui de la figure 1 pour lequel les numéros de référence ont été augmentés de 100. Ainsi, l'élément support porte le numéro de référence 108, la membrane le numéro de référence 106 et la bague de serrage le numéro de référence 114. On observera ici que l'élément support 108 présente une forme cylindrique-conique fermée à son extrémité inférieure 108a par un élément d'obturation 118 qui est avantageusement également perforable pour permettre le remplissage du réservoir 2. On peut prévoir que l'extrémité inférieure 108a est cassable grâce à la prévision d'une prédécoupe annulaire 130 de manière à permettre la fixation d'une aiguille d'injection intradermique standard du commerce 140 dont l'embase 142 vient se fixer sur l'élément support 108. Ce mode de réalisation présente l'avantage d'utiliser ainsi une aiguille d'injection intradermique standard du commerce.

Selon le troisième mode de réalisation représenté à la figure 3, le dispositif thérapeutique selon l'invention 201 comprend un élément support 208 de forme sensiblement cylindrique creuse, ou en disque, comprenant une cavité 209 dans laquelle est montée la membrane déformable 206 de manière à subdiviser cette cavité 209 en deux compartiments distincts ou non communicants 209a, 209b.

Dans la position de repos, de non-déformation de la membrane déformable 206, qui est celle représentée à la figure 3 en trait plein, le compartiment 209b qui est destiné à constituer le réservoir de solution liquide d'agent thérapeutique a ici un volume sensiblement nul, la membrane 206 reposant ici sur la partie frontale 208a de l'élément support 208. Cette partie frontale, qui est destinée à être appliquée contre la peau d'un être vivant, est pourvue d'un organe 220 d'injection transcutanée, qui est ici monté à demeure dans la partie frontale 208a qui présente une épaisseur de paroi relativement grande pour tenir fermement cet organe d'injection transcutanée 220.

Par ailleurs, on prévoit avantageusement des moyens ou organes de remplissage séparés ou distincts 226 comprenant par exemple un orifice 228 obturé par un moyen d'obturation perforable 229 qui peut être ici formé par un bouchon disposé dans un épaulement affleurant la partie frontale 208a de manière à être facilement enlevable.

Par ailleurs, on peut prévoir que la partie opposée 208b de l'élément support 208 présente une section transversale sensiblement en U à fond plat pourvue d'un épaulement interne 232 contre lequel est appliquée la membrane déformable 206 qui y est maintenue fermement lors de la fixation de la partie frontale 208a, par des moyens de fixation appropriés 236, tels que filetages.

Le compartiment 209a qui est défini entre la membrane déformable 206 et la partie en U 208b peut être mis sous vide et on prévoit dans ce cas un orifice 242 de mise en communication avec l'atmosphère par la destruction de l'opercule 244 comme symbolisé par les pointillés 246.

On comprend ainsi que, si l'on introduit par injection forcée la substance liquide contenant le ou les agents thérapeutiques par perforation de l'élément d'obturation 229 perforable, on va produire une déformation de la membrane déformable 206 par exemple jusqu'à la position indiquée en traits mixtes à la figure 3, ceci étant facilité par la mise sous vide du compartiment 209a, en créant ainsi le compartiment 209b constituant le réservoir de solution liquide à injecter par voie transcutanée, analogue au réservoir 2 des figures 1 et 2.

En outre, par destruction de l'opercule 244, la pression atmosphérique est rétablie dans le compartiment 209a, de sorte que la membrane déformable 206, qui est aussi prévue élastique, qui était préalablement précontrainte par le vide régnant dans le compartiment 209a, d'une part, et la pression exercée par la présence de la solution liquide dans le compartiment 209b, d'autre part, est libérée de cette précontrainte et exerce alors une poussée sur la composition liquide d'agent thérapeutique présent dans le compartiment 209b, cette poussée étant proportionnelle à la précontrainte qui elle-même est fonction de l'élasticité de la membrane.

On peut ainsi moduler à volonté la période de temps nécessaire pour injecter ou faire écouler complètement la solution contenant l'agent thérapeutique désiré par l'organe d'injection transcutanée 220. De même, on peut prévoir une couche d'adhésif 238 protégée par une bande de protection détachable non représentée ici.

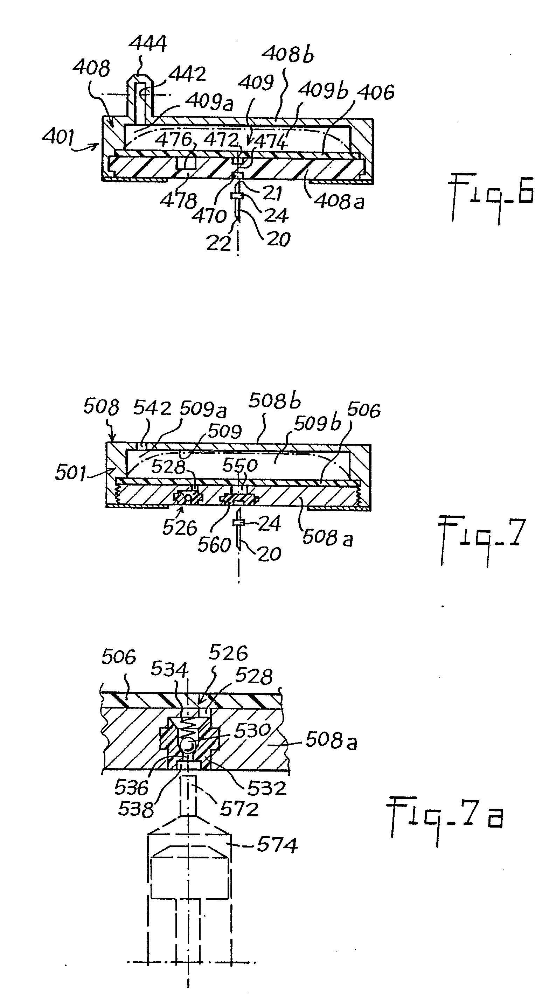

En référence à la figure 4, on a représenté une variante de réalisation du mode de réalisation de la figure 3 selon laquelle l'élément d'obturation 229' est ici monté à demeure dans la partie frontale 208'a.

En référence à la figure 5, on a représenté un autre mode de réalisation constituant une variante de réalisation du mode de réalisation de la figure 3 pour lequel les numéros de référence ont encore été augmentés de 100. Selon ce mode de réalisation, au lieu d'avoir l'organe d'injection transcutanée 220 monté à demeure, on prévoit dans la paroi frontale 308a un orifice 350 obturé par un élément d'obturation 360 qui peut être monté à demeure ou être monté de manière détachable.

Selon cette réalisation, on peut faire le vide dans les deux compartiments 309a, 309b, qui sont étanches.

Cet élément d'obturation perforable 360 peut être perforé par une aiguille creuse identique à l'aiguille 20 de la figure 1.

Par cet orifice 350, on peut alors introduire la solution liquide d'agent thérapeutique à injecter et réaliser son injection par l'organe d'injection transcutanée 20. Mais, on peut aussi prévoir un orifice séparé 328 pour l'injection obturé par l'élément d'obturation 326 comme dans le cas du mode de réalisation de la figure 3 après avoir perforé l'élément 360 par l'aiguille 20.

Le fonctionnement de ce dispositif est identique à celui de la figure 3 après avoir perforé l'élément 360 par l'aiguille 20.

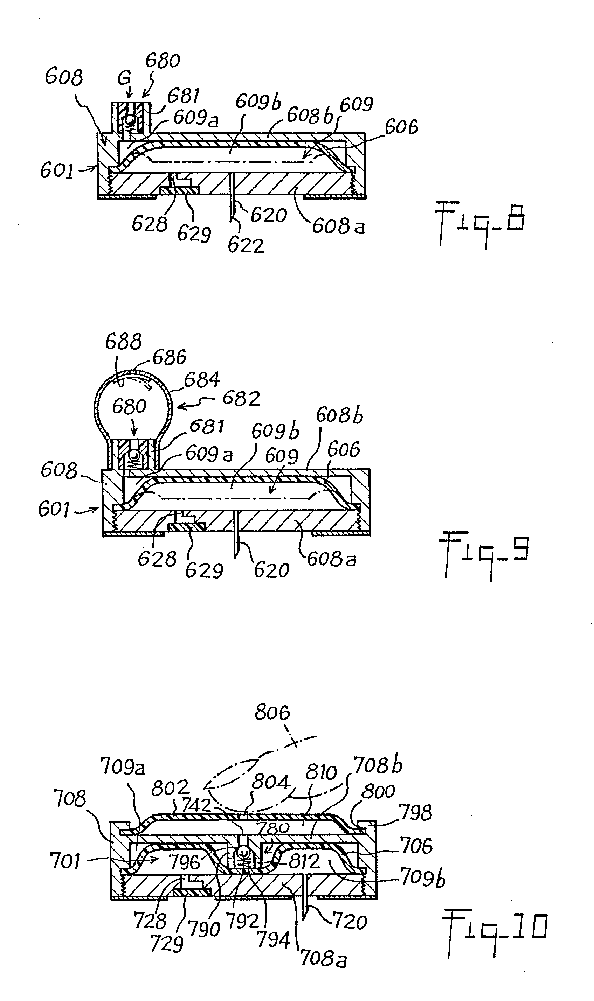

En référence à la figure 6, on a représenté encore un autre mode de réalisation constituant une variante de réalisation de celui des figures 3 et 5, pour laquelle les numéros de référence ont encore été augmentés de 100. Cette variante de réalisation consiste à prévoir la partie frontale 408a de l'élément support 408 constitué en un matériau étanche perforable, tel que caoutchouc.

Ainsi, on prévoit par exemple au moins une prédécoupe 470, 472 ayant pour but de réduire l'épaisseur de la partie frontale 408a dans un endroit prédéterminé où est prévue la fixation de l'organe d'injection 20 identique à celui des figures 1 et 5 par perforation de la partie de cloison amincie 474.

Cette perforation de paroi amincie 474 peut remplir le double rôle de permettre l'introduction de la solution liquide d'agent thérapeutique dans le compartiment 409b défini par la membrane 406 de la partie frontale 408a et ensuite l'injection transcutanée lorsque l'on aura fixé par perforation l'organe d'injection 20.

Cependant, on peut prévoir à un autre endroit au moins une autre rainure ou découpe de paroi 476 réalisant un nouvel amincissement 478 de la partie frontale 408a permettant l'introduction de la solution liquide d'agent thérapeutique tandis que l'injection est réalisée par perforation de l'autre partie amincie 474.

On comprend que le mode de fonctionnement de ce mode de réalisation soit identique à ceux des figures 3 et 5.

En référence à la figure 7, on a représenté un autre mode de réalisation qui constitue encore une variante de réalisation relativement aux modes de réalisation des figures 5 et 6, pour laquelle les numéros de référence ont encore été augmentés de 100 pour les pièces similaires.

Selon cette variante de réalisation, la partie frontale 508a comporte au moins un orifice 550 traversant la paroi frontale 508a de part en part, obturé par un élément d'obturation 560 pouvant comporter une partie de paroi centrale amincie lui conférant ainsi une forme en U, comme l'élément 360 (figure 5). Cet élément d'obturation est perforable et peut être réalisé par exemple en caoutchouc. Il peut également remplir la double fonction d'introduction de la solution liquide d'agent thérapeutique dans le compartiment 509b défini entre la membrane 506 de la partie frontale 508a et d'injection transcutanée lorsque l'organe d'injection 20 aura été également introduit par perforation de cet élément d'obturation 560.

On peut aussi bien selon une autre variante, telle que représentée, prévoir un orifice distinct 528, obturé au moins temporairement par un élément d'obturation 526 qui est représenté plus en détail, de manière agrandie à la figure 7a. Cet élément d'obturation 526 forme ici avantageusement un clapet antiretour constitué par une valve à bille 530 maintenue en permanence contre un siège de valve 532 par un moyen de rappel élastique 534 ayant tendance à maintenir en permanence la bille 530 contre son siège 532. L'élément formant siège 532 peut être réalisé en caoutchouc et comporte des orifices 536, 538 permettant le positionnement de l'extrémité 572 d'une seringue 574 représentée en traits interrompus à la figure 7a pour l'introduction forcée de la solution liquide d'agent thérapeutique jusque dans le compartiment 509b défini entre la membrane 506 et la partie frontale 508a, ce compartiment 509b étant créé au fur et à mesure de l'introduction de la solution liquide d'agent thérapeutique par déformation de la membrane 506, comme précédemment décrit en détail relativement au mode de réalisation de la figure 3 notamment.

On observera que, également selon cette variante, le compartiment 509a communique librement avec l'extérieur par un orifice 542 similaire à l'orifice 242 du mode de réalisation de la figure 3.

On comprend ainsi que, lorsque la membrane déformable 506 aura été déformée par l'introduction forcée de la solution liquide d'agent thérapeutique et prendra la position déformée représentée en traits mixtes à la figure 7, la pression atmosphérique qui s'exerce dans le compartiment 509a communique librement avec l'atmosphère par l'orifice 542, de sorte que la membrane 506 va venir progressivement reprendre sa position initiale non déformée présentée en trait plein, contre la partie frontale 508a grâce à là quantité d'énergie emmagasinée dans la membrane 506 à l'état déformé et à l'action de la pression atmosphérique dans le compartiment 509a, en provoquant ainsi progressivement l'injection transcutanée de la solution liquide d'agent thérapeutique, d'une manière tout à fait similaire aux modes de réalisation des figures 1 et 2 et également des modes de réalisation des figures 3 à 6 après enlèvement de l'opercule respectivement 244, 344 ou 444.

En référence à la figure 8, on a représenté encore un autre mode de réalisation d'un dispositif thérapeutique selon l'invention constituant une variante de réalisation de celui de la figure 3 pour lequel on a utilisé les mêmes numéros de référence mais encore augmentés de 100 par rapport au mode de réalisation de la figure 7.

On retrouve ainsi l'organe d'injection transcutanée 620 fixé en permanence dans la partie frontale 608a, l'orifice d'introduction 628 obturé par un élément d'obturation perforable 629, le compartiment 609a dans lequel on exercera une pression contre la membrane 606 supérieure à la pression régnant dans le compartiment 609b situé entre la membrane 606 et la partie frontale 608a lorsqu'il contiendra la solution liquide d'agent thérapeutique à injecter par voie transcutanée.

Dans cette variante de réalisation, la membrane 606 déformable présente à l'origine une forme en coupelle inversée ou en U inversé à fond plat venant reposer contre la paroi de la partie 608b de l'élément support 608. Ici, les compartiments 609a et 609b sont habituellement à la pression atmosphérique.

Le fonctionnement de ce mode de réalisation découle clairement du fonctionnement des autres modes de réalisation et consiste tout d'abord à remplir le compartiment 609b avec la solution liquide d'agent thérapeutique par perforation de l'élément d'obturation perforable 628.

Ensuite, on exerce une surpression dans le compartiment 609a par l'introduction d'un gaz G par l'intermédiaire de l'élément formant valve 680 à clapet antiretour, de structure similaire à celle de la valve 526 décrite en détail à la figure 7a. Lorsque l'on libère l'ouverture de l'extrémité libre 622 de l'organe 620 et que l'on applique le dispositif sur la peau d'un patient de manière à réaliser l'injection transcutanée, la membrane 606 peut être progressivement déformée en direction de la partie frontale 608a, en réduisant ainsi le volume du compartiment 609b, ce qui va provoquer l'injection transcutanée de la solution liquide d'agent thérapeutique.

En référence à la figure 9, on a représenté une variante de réalisation du mode de réalisation de la figure 8 pour laquelle on a utilisé les mêmes numéros de référence pour les parties identiques. On observa ici que l'on a simplement rajouté un dispositif de gonflage 682 sur l'embout annulaire 681 dans lequel est montée la valve. Ce dispositif de gonflage 682 peut être en pratique constitué par une simple poire en caoutchouc 684 munie d'un système 686, 688 d'aspiration-refoulement permettant de réaliser aisément et économiquement la surpression dans le compartiment 609a. En outre, cette variante offre l'avantage de permettre au patient d'actionner lui-même le dispositif de gonflage à intervalle de temps défini, afin de relancer l'injection de l'agent thérapeutique contenu dans le compartiment 609b.

En référence à la figure 10, on a représenté encore une variante de réalisation des modes de réalisation des figures 8 et 9 pour laquelle les numéros de référence ont encore été augmentés de 100. Ainsi, l'élément support porte le numéro de référence 708, la membrane déformable le numéro de référence 706, la partie frontale le numéro de référence 708a. Dans cette variante de réalisation, la partie arrière de paroi 708b de l'élément support 708 comporte une partie de paroi centrale de forme cylindrique 790 creuse faisant saillie intérieurement dans le compartiment 709b jusqu'à venir appliquer la partie centrale de membrane 706 contre la paroi frontale 708a. Le compartiment 709a présente alors une forme annulaire et la membrane 706 la forme particulière représentée à la figure 10 en position de repos. La partie centrale cylindrique 790 creuse de la partie arrière 708b forme également siège pour une bille 792 poussée en permanence par un moyen de rappel 794 contre le siège 796 de la partie cylindrique 790 de manière à empêcher au moins temporairement une communication entre le compartiment 709a par les orifices 812 et l'extérieur par l'orifice 742, comme clairement compréhensible.

Selon cette variante, l'élément support 708 comporte latéralement un épaulement annulaire 798, situé dans le prolongement du pourtour de l'élément support 708 et faisant saillie extérieurement par rapport au plan de la partie arrière 708b. Cet épaulement annulaire 798 comporte une encoche annulaire 800 permettant l'insertion d'une nouvelle membrane 802 comportant un orifice central 804 pouvant être obturé par exemple par le doigt 806 d'un opérateur, en général le praticien ou le patient lui-même.

On comprend qu'avec ce mode de réalisation de la figure 10 on crée un compartiment 810 entre la membrane supplémentaire 802 et la paroi arrière 708b de l'élément support 708 qui sera habituellement en communication avec l'atmosphère. Ainsi, lorsqu'un opérateur va poser son doigt 806 contre l'orifice 804 et exercera une pression sur la membrane 802 de manière à l'appliquer contre la partie arrière 708b de l'élément support 708, il se produira une injection d'air par l'orifice 742 repoussant la bille 792 fonctionnant comme une valve, cet air étant ensuite introduit dans le compartiment 709b par les orifices 812 prévus dans la partie cylindrique 790, en exerçant alors une surpression contre la membrane déformable 706 en diminuant le volume du compartiment 709a et en provoquant ainsi l'injection transcutanée de la solution liquide préalablement introduite dans le compartiment 709a par perforation de l'élément d'obturation perforable 729.

On comprend ainsi que tous les modes de réalisation précédemment décrits fonctionnent sur le même principe de base qui consiste à exercer une pression telle sur la membrane déformable que celle-ci va réduire le volume du compartiment contenant la solution liquide d'agent thérapeutique qui sera alors injectée progressivement par voie transcutanée. Cette membrane déformable peut être déformée au préalable par l'introduction même de la solution liquide d'agent thérapeutique dans le compartiment formant réservoir destiné à la contenir, ce qui est prévu dans les modes de réalisation des figures 1 à 7a incluse. L'injection transcutanée est alors provoquée par la pression exercée par la membrane qui a tendance à vouloir reprendre sa forme initiale et par la pression atmosphérique environnante s'exerçant sur la membrane, soit en permanence comme dans le cas des figures 1, 2 et 7, 7a, soit au moment de l'injection par destruction d'un opercule de mise en communication avec l'atmosphère comme dans le cas des modes de réalisation des figures 3; 4 ; 5 ; 6.

Dans tous ces modes de réalisation des figures 1 à 7a, le compartiment destiné à contenir la solution liquide d'agent thérapeutique présente au départ un volume réduit ou nul comme cela est clairement compréhensible à partir de la considération de ces figures. Par contre, selon les variantes de réalisation des figures 8 à 10, le volume du compartiment destiné à contenir la solution liquide d'agent thérapeutique est réalisé à l'origine par une conformation particulière de la membrane déformable. Dans ce cas, la déformation de la membrane, réduisant le volume du compartiment contenant la solution liquide d'agent thérapeutique, est obtenue en exerçant une surpression contre celle-ci par l'introduction d'un gaz approprié, qui est de préférence constitué simplement par de l'air.

On comprend ainsi que l'on obtient tous les avantages techniques précédemment décrits et la mise en oeuvre du procédé de fabrication précédemment énoncé qui n'est pas répété ici.

Naturellement, l'invention comprend tous les moyens constituant des équivalents techniques des moyens décrits ainsi que leurs diverses combinaisons. En particulier, on peut selon une variante prévoir que la membrane soit doublée,du côté du compartiment contenant la solution liquide d'agent thérapeutique à injecter, d'une deuxième membrane souple, déformable, réalisée en un matériau chimiquement compatible avec l'agent thérapeutique à injecter et assurant éventuellement l'imperméabilité de la membrane, cette membrane pouvant être constituée par exemple par du téréphtalate de polyéthylène.

**Revendications**

1. Procédé de fabrication d'un dispositif thérapeutique d'injection transcutanée portable, totalement autonome, comprenant un élément formant réservoir d'un agent à activité thérapeutique à injecter par voie transcutanée, caractérisé en ce qu'on prévoit au moins un organe d'injection transcutanée, par exemple une aiguille, des moyens pour provoquer l'injection transcutanée de ladite solution depuis ledit réservoir; de préférence, on prévoit une membrane déformable (6 ; 106 ; 206 ; 306 ; 406 ; 506 ; 606 ; 706) comme moyen provoquant l'injection de ladite solution contenue dans ledit réservoir ; et on monte ladite membrane sur ou dans le dispositif thérapeutique de manière à permettre un mouvement de la membrane déformable réduisant le volume du réservoir d'agent thérapeutique, notamment lorsqu'on exerce sur la membrane, en direction de l'organe d'injection, une pression supérieure à la pression régnant dans le réservoir, pour provoquer alors ladite injection transcutanée.

2. Procédé selon la revendication 1, caractérisé en ce qu'on monte la membrane déformable à l'état non déformé, en l'absence d'agent thérapeutique, de sorte que ledit réservoir est d'un volume réduit, voire sensiblement nul, cette membrane étant montée de manière à pouvoir être déformée à un moment quelconque avant l'injection transcutanée avantageusement par l'introduction forcée de la solution liquide d'agent thérapeutique dans le réservoir, cette déformation étant prévue pour permettre d'emmagasiner une quantité d'énergie dans la membrane suffisante pour permettre l'injection progressive transcutanée de ladite solution d'agent thérapeutique lorsque la membrane reprend progressivement sa forme initiale non déformée.

3. Procédé selon la revendication 1, caractérisé en ce que le dispositif thérapeutique comprend une cavité dans laquelle on dispose la membrane déformable précitée de manière à subdiviser la cavité en deux compartiments distincts non communicants, un compartiment constituant le réservoir de solution liquide d'agent thérapeutique précité, l'autre compartiment étant prévu pour pouvoir exercer contre la membrane, de préférence à l'aide d'un gaz introduit sous pression, une pression supérieure à la pression régnant dans le réservoir contenant l'agent thérapeutique.

4. Dispositif thérapeutique d'injection transcutanée, portable, totalement autonome, comprenant un élément formant réservoir pour la réception d'une solution liquide d'au moins un agent à activité thérapeutique, caractérisé en ce qu'il est prévu pour recevoir au moins un organe d'injection transcutanée, par exemple une aiguille ou une pointe, et des moyens pour provoquer l'injection de ladite solution liquide depuis ledit réservoir, de préférence, il comprend une membrane déformable (6 ; 106 ; 206 ; 306 ; 406 ; 506 ; 606 ; 706) comme moyen provoquant l'injection de ladite solution ; et des moyens permettant un déplacement de la membrane réduisant le volume dudit réservoir en provoquant une injection transcutanée par ledit organe (20 ; 140 ; 220 ; 620 ; 720).

5. Dispositif thérapeutique selon la revendication 4, caractérisé en ce que la membrane déformable précitée (6 ; 106 ; 206 ; 306 ; 406 ; 506) est déformée à un moment quelconque avant l'injection, cette membrane déformable ainsi déformée constituant alors des moyens exerçant sur la membrane une pression supérieure à celle régnant dans le réservoir.

6. Dispositif thérapeutique selon la revendication 4 ou 5, caractérisé en ce que la membrane déformable précitée (6 ; 106 ; 206 ; 306 ; 406 ; 506 ; 606 ; 706) forme au moins une partie du réservoir d'agent thérapeutique.

7. Dispositif thérapeutique selon une des revendications 4 à 6, caractérisé en ce que la membrane déformable précitée est montée sur un élément support (8 ; 108 ; 208 ; 308 ; 408 ; 508 ; 608 ; 708) avantageusement pourvu d'un organe de remplissage (16-18 ; 116-118 ; 226 ; 326-328 ; 470-472-474 ; 550-560 ; 628-629 ; 628-729) dudit réservoir, avantageusement cet élément support de membrane comporte une partie de paroi définissant avec ladite membrane le réservoir précité.

8. Dispositif thérapeutique selon la revendication 7, caractérisé en ce que l'élément support (8 ; 108 ; 208 ; 308 ; 408 ; 508 ; 608 ; 708) a en coupe au moins partiellement une forme sensiblement cylindrique creuse.

9. Dispositif thérapeutique selon la revendication 8, caractérisé en ce que l'élément support (8 ; 108) comprend vers une extrémité un épaulement annulaire (10 ; 110) radialement saillant vers l'extérieur contre lequel est maintenu solidement le bord (12 ; 112) de la membrane (8 ; 108) par un moyen de maintien approprié (14 ; 114), tel qu'une bague de serrage.

10. Dispositif thérapeutique selon l'une des revendications 4 à 9, caractérisé en ce que le dispositif comprend une cavité (209 ; 309 ; 409 ; 509 ; 609 ; 709) dans laquelle est montée la membrane précitée de manière à subdiviser cette cavité en deux compartiments distincts non communicants (respectivement 209a, 209b ; 309a, 309b ; 409a, 409b ; 509a, 509b ; 609a, 609b ; 709a, 709b) dont un compartiment (209b, 309b, 409b, 509b, 609b, 709b) constitue le réservoir précité destiné à contenir la solution liquide d'agent thérapeutique, et l'autre compartiment (209a ; 309a ; 409a ; 509a ; 609a ; 709a) est prévu pour pouvoir exercer contre la membrane, de préférence à l'aide d'un gaz introduit sous pression, une pression supérieure à la pression régnant dans le réservoir contenant la solution liquide d'agent thérapeutique.

11. Dispositif thérapeutique selon la revendication 10, caractérisé en ce que le compartiment (209a ; 309a ; 409a) distinct du compartiment (209b ; 309b ; 409b ; 509b) contenant la solution liquide d'agent thérapeutique est sous vide, la paroi arrière (208b ; 308b ; 408b ; 508b) de l'élément support, opposée à la partie frontale (208a ; 308a ; 408a) qui est pourvue de l'organe d'injection, est pourvue d'un orifice (242 ; 342 ; 442) operculé de mise en communication avec l'atmosphère par destruction d'un opercule (244 ; 344 ; 444).

12. Dispositif thérapeutique selon la revendication 11, caractérisé en ce que la partie arrière (508b) de l'élément support (508) commune avec le compartiment (509a) destiné à exercer une pression sur la membrane (506) est pourvue d'un orifice (542) de communication libre avec l'atmosphère.

13. Dispositif thérapeutique selon la revendication 10, caractérisé en ce que la partie arrière (108b ; 708b) de l'élément support (508 ; 608 ; 708) comprend des moyens de mise en surpression du compartiment (609a ; 709a), cette surpression étant établie au moment de l'utilisation du dispositif ou à tout autre moment, ces moyens de mise en surpression pouvant comprendre un système de valve de gonflage (680 ; 780).

14. Dispositif thérapeutique selon l'une quelconque des revendications précédentes, caractérisé en ce que l'organe de remplissage du réservoir précité comprend un orifice (16 ; 116 ; 228 ; 328 ; 528 ; 628 ; 728) coopérant avec un élément d'obturation avantageusement perforable (18 ; 118 ; 229 ; 326 ; 629 ; 729).

15. Dispositif thérapeutique selon la revendication 13, caractérisé en ce que l'organe de remplissage comprend un dispositif à valve de remplissage (526).

16. Dispositif thérapeutique selon l'une quelconque des revendications 4 à 15, caractérisé en ce que la membrane élastique (6 ; 106 ; 206 ; 306 ; 406 ; 506 ; 606 ; 706) est isolée de tout contact avec l'agent thérapeutique contenu dans le compartiment (2 ; 209b ; 309b ; 409b ; 509b ; 609b ; 709b) par l'intermédiaire d'une deuxième membrane déformable, élastique ou non, étanche et chimiquémemt compatible avec la solution liquide d'agent thérapeutique.

17. Dispositif thérapeutique selon l'une quelconque des revendications 4 à 16, caractérisé en ce qu'il est réalisé sous une forme stérile et avantageusement jetable, en totalité ou en partie, le remplissage du réservoir étant de préférence réalisé après le conditionnement stérile.

18. Dispositif thérapeutique selon l'une quelconque des revendications 4 à 17, caractérisé en ce qu'il est réalisé sans agent thérapeutique dans le réservoir, le praticien effectuant lui-même le remplissage par l'organe de remplissage précité avec le mélange de son choix et sa composition.

19. Dispositif thérapeutique selon l'une quelconque des revendications 4 à 18, caractérisé en ce qu'il présente la forme d'une pastille ou d'une bande, avantageusement pourvue d'un adhésif faisant ou non partie du dispositif pour être maintenue par adhésion sur la peau d'un être vivant, cet adhésif étant avantageusement provisoirement protégé par une couche de protection détachable qui englobe l'organe d'injection transcutanée lorsque celui-ci est présent dès l'origine sur le dispositif.

0258073

Fig-1

Fig-2

Fig-3

Fig-4

Fig-5

0258073

Fig-6

Fig-7

Fig-7a

0258073

Fig-8

Fig-9

Fig-10

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| X | US-A-4 386 929 (ALZA)<br><br>* Description; figures * | 1,2,4, 5,7-9, 18 | A 61 M 5/20<br>A 61 M 5/14 |
| X | US-A-4 552 561 (ECKENHOFF)<br><br>* Colonne 3, ligne 24 - colonne 5, ligne 11; figures 2,8,13 * | 1,3-6, 10,13, 14,17-19 | |
| X | US-A-3 468 308 (BIERMAN)<br><br>* Colonne 6, ligne 70 - colonne 7, ligne 62; colonne 4, lignes 57-69; figures 1,3,11,13 * | 1-4,7-11,18 | |
| X | US-A-4 340 048 (ALZA)<br><br>* Colonne 5, ligne 39 - colonne 6, ligne 31; figure 4 * | 1,3-8, 10,14, 17-19 | DOMAINES TECHNIQUES RECHERCHES (Int Cl.4)<br><br>A 61 M |
| X | FR-A-2 091 189 (UNIVERSITY OF MINNESOTA)<br>* Description; figures * | 1,3-10 ,13,14 | |

Le present rapport de recherche a été établi pour toutes les revendications

| Lieu de la recherche | Date d'achèvement de la recherche | Examinateur |
|---|---|---|
| LA HAYE | 25-09-1987 | VANRUNXT J.M.A. |